# EUROPEAN PATENT APPLICATION

(11) **EP 2 799 425 A1**
(43) Date of publication of application: **05.11.2014**
(21) Application number: 13382158.7
(22) Date of filing: 29.04.2013
(51) Int. Cl.: C07C 205/59, C07C 201/12, C07D 231/22, C07C 227/04, C07C 249/16

(54) **Preparation process of an agonist of the thrombopoietin receptor**

(71) Applicant: Esteve Química, S.A., 08024 Barcelona (ES); Galchimia, S.L., 15823 O Pino (ES)
(72) Inventor: Bartra Sanmarti, Martí, 08024 Barcelona (ES); Solsona Rocabert, Joan Gabriel, 08024 Barcelona (ES); Cruces Colado, Jacobo, 15823 O Pino (ES); Enjo Babio, Juan, 15823 O Pino (ES); Pampin Casal, María Begoña, 15823 O Pino (ES)
(74) Representative: ZBM Patents - Zea, Barlocci & Markvardsen

(57) **Abstract**

It relates to a preparation process of 2'-(benzyloxy)-3'-nitro-1,1'-biphenyl-3-carboxylic acid or a salt thereof comprising reacting 2-(benzyloxy)-1-bromo-3-nitrobenzene with a either 3-carboxyphenylboronic acid or a salt thereof or a (C₁-C₄)-alkyl ester thereof in the presence of Pd(OAc)₂, tricyclohexylphosphine, a base, an appropriate solvent, and at an appropriate temperature; if appropriate, submitting the compound thus obtained to a hydrolysis reaction; and isolating the compound thus obtained in form of a salt of compound of formula (VI) or in form of the free acid by adding an acid. It also comprises the further preparation to eltrombopag or its salts from the new intermediate thus obtained by subsequent reduction of the nitro group and deprotection of the phenol, conversion of the amine intermediate obtained in a diazonium derivative, and either (1) subsequent reaction with ethyl acetoacetate and with (3,4-dimethylphenyl)hydrazine or a salt thereof, occurring the pyrazole ring formation by intermolecular cyclization, or (2) introduction of the pyrazole ring by reaction with 1-(3,4-dimethylphenyl)-3-methyl-3-pyrazolin-5-one.

## Description

The invention refers to a process for the preparation of an active pharmaceutical ingredient known as eltrombopag and salts thereof, as well as some intermediates useful in this preparation process.

### BACKGROUND ART

Eltrombopag olamine is the International Non-proprietary Name (INN) of 3'-[(2Z)-1-(3,4-dimethylphenyl)-1,5-dihydro-3-methyl-5-oxo-4*H*-pyrazol-4-ylidene]hydrazinyl]-2'-hydroxy-[1,1'-biphenyl]-3-carboxylic acid, compound with 2-aminoethanol (1:2) , and has a CAS No. 496775-62-3. Eltrombopag olamine is indicated for the treatment of thrombocytopenia in patients with chronic immune (idiopathic) thrombocytopenic purpura. It is marketed under the trade name Promacta® in USA by GlaxoSmithKIine.

The structure of eltrombopag olamine corresponds to the following formula:

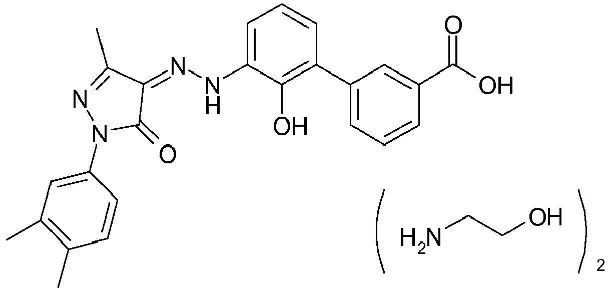

International patent application WO200189457A2 discloses several processes to prepare certain hydroxy-1-azo-benzene derivatives which are mimetic compounds of thrombopoietin. One of these processes relates to the preparation of a group of compounds encompassed in a Markush formula which includes the eltrombopag. It is disclosed in a generic way the formation of 3'-amino-2'-hydroxy-1,1'-biphenyl-3-carboxylic acid derivatives from the corresponding 2-bromo-6-nitrophenol derivatives by protection of the phenol group and subsequent Suzuki reaction. The hydroxyl protective group may be an alkyl group such as methyl or an alkyl substituted group such as benzyl, and may be introduced by reaction with an alkylating agent such as methyl iodide or benzyl bromide, in the presence of a base such as sodium hydride or potassium carbonate, and a suitable solvent such as dimethylformamide, tetrahydrofuran, or acetone, to give the corresponding protected nitrophenol compound. However, it is only exemplified the process that goes through the protection of the phenol as methoxide (cf. for instance, Example 3 for the preparation of 3'-amino-2'-hydroxy-1,1'-biphenyl-3-carboxylic acid). Unfortunately, the protection as methoxide implies the use of methyl iodide and the formation of methyl bromide in the demethylation reaction, both compounds being highly toxic reactives which are not appropriate at industrial scale. In addition, drastic deprotection reaction conditions are disclosed as well as high dilution rates in the subsequent transformation of the compound obtained to the corresponding amino derivative. Subsequently, the nitro compound is reduced to give the corresponding amino compound.

The inventors have experimentally confirmed that applying the Suzuki reaction in the described conditions for the methoxide protected compound (cf. example 3c with tetrakistriphenylphosphino palladium (0) as catalyst) to the benzyloxy protected compound yields to an nitro benzyloxybiphenyl compound with a low yield (∼37%) and which is unpurified with triphenylphosphine oxide, being difficult to be removed.

On the other hand, the known process for the preparation of eltrombopag comprises the introduction of the pyrazol ring in a single reaction step by conversion of the 3'-amino-2'-hydroxy-1,1'-biphenyl-3-carboxylic acid in a diazonium derivative and subsequent coupling with the corresponding pyrazol derivative (cf. Example 3 of WO01/89457A2, yield 32%). The pyrazol derivative is previously prepared by condensation of the corresponding phenyl hydrazine derivative with ethyl acetoacetate. Likewise, Example 1 of WO2010114943A1 discloses the same coupling between the amino precursor and the pyrazol derivative in slightly different conditions with a yield of 95.4%. It has experimentally confirmed that the efficacy of the process, in terms of yield and also of purity, is highly dependent on the quality of the starting materials, being highly variable.

Finally, European patent application EP2152811A1 discloses an alternative process for the preparation of analogous compounds to eltrombopag based on the use of a phenol derivative which is not covalently protected but used as addition compound with a primary amine. This compound is reacted with the corresponding boronic compound, and then the pyrazole ring is introduced in a single step.

Despite the teaching of all these prior art documents, the research of new preparation processes of eltrombopag is still an active field, since the industrial exploitation of known processes is difficult, as it has been pointed out above. Thus, the provision of new preparation processes of eltrombopag is desirable.

### SUMMARY OF THE INVENTION

Inventors have found an improved process for the preparation of an intermediate compound for the preparation of eltrombopag, 3'-amino-2'-hydroxy-1,1'-biphenyl-3-carboxylic acid, which avoids the problems found in the known processes and allows obtaining the previous compound with high yield. They can be obtained also with high purity. The process comprises the preparation of this intermediate through a benzylic protected intermediate compound, applying specific reaction conditions and specific reactants in the Suzuki reaction not suggested in the prior art, followed by subsequent reduction of the nitro group and deprotection of the phenol group in a single step, making the process more appropriate for industrial scale.

Accordingly an aspect of the present invention relates to a preparation process of a compound of formula (VI) or a salt thereof, 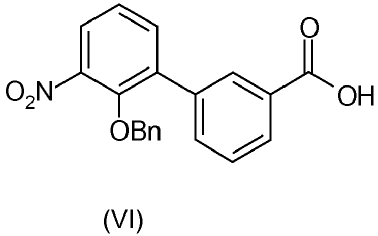 comprising reacting a compound of formula (VII) with either a compound of formula (VIII) or a salt thereof or a (C₁-C₄)-alkyl ester thereof, in the presence of Pd(OAc)₂, tricyclohexylphosphine, a base, an appropriate solvent, and at an appropriate temperature; if appropriate, submitting the compound thus obtained to a hydrolysis reaction to transform the ester moiety to a carboxyl moiety; and isolating the compound thus obtained in form of a salt of compound of formula (VI) or in form of the free acid by adding an acid; 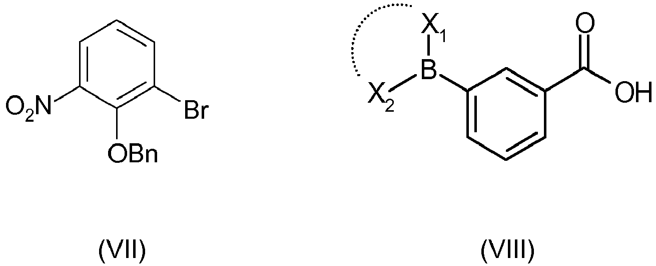 wherein Bn is a benzyl group (C₆H₅CH₂-); X₁ and X₂ are each independently selected from the group consisting of hydroxy, (C₁-C₄)-alcoxy and phenoxy, the latter optionally substituted by a (C₁-C₄)-alcoxy or a (C₁-C₄)-alkyl; or alternatively X₁ and X₂ can be taken together with the boron atom to form a cyclic structure selected from the following ones,

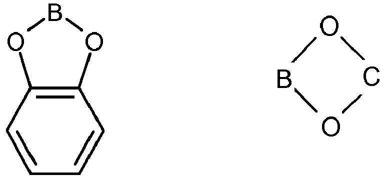

C is (CH₂)ₙ wherein the hydrogen atoms of the CH₂ group are optionally replaced by a substituent selected from (C₁-C₄)-alkyl, phenyl and mono- or disubstituted phenyl, the substituents being (C₁-C₄)-alkyl and (C₁-C₄)-alkoxy, and n is an integer from 2 to 4.

The compound of formula (VI) in form of free acid may be submitted to a reduction and deprotection reaction in a single step using H₂ and a palladium catalyst to yield 3'-amino-2'-hydroxy-1,1'-biphenyl-3-carboxylic acid of formula (IV). The reaction may be carried out in the presence of an appropriate base, for instance, sodium hydroxide. The compound (VI) may be used in form of a salt and the reaction can be carried out in the presence of an appropriate base.

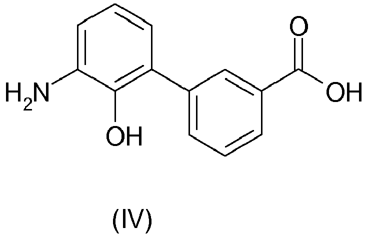

The compound of formula (IV) may be converted into a salt thereof by reaction with a base or acid.

The compound of formula (IV) or a salt thereof obtained following the process disclosed above may be converted into eltrombopag acid or a salt thereof by reacting the compound of formula (IV) or a salt thereof with sodium nitrite to form the corresponding diazonium derivative and then with 1-(3,4-dimethylphenyl)-3-methyl-3-pyrazolin-5-one of formula (IX).

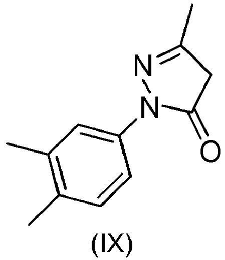

Optionally, the eltrombopag thus obtained may be reacted with an appropriate pharmaceutically acceptable base to yield a pharmaceutically acceptable salt of eltrombopag. Therefore, a process comprising the steps for preparing eltrombopag or its salts from the compound of formula (VII) as disclosed above is part of the invention.

On the other hand, the inventors have been developed another process for the transformation of the compound of formula (IV) or a salt thereof to eltrombopag acid or a salt thereof. The process comprises the formation of the pyrazole ring in two steps.

In a first step the amine derivative is converted into a diazonium derivative by reaction with sodium nitrite and then reacted with ethyl acetoacetate to yield a compound of formula (II) or a salt thereof.

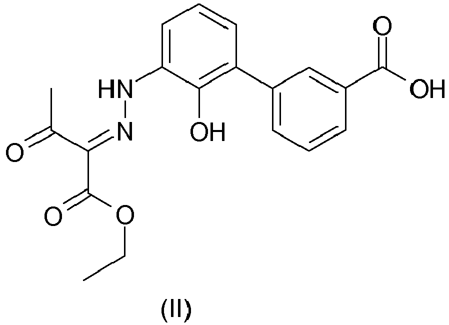

In a second step, the compound thus obtained is condensed with (3,4-dimethylphenyl)hydrazine of formula (III) or a salt thereof, in particular the hydrochloride salt, occurring the pyrazole ring formation by intermolecular cyclization.

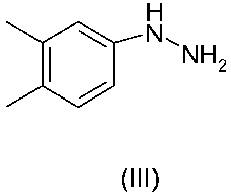

This step is carried out in the presence of an acid, and an appropriate solvent, and yields to eltrombopag free acid of formula (I). 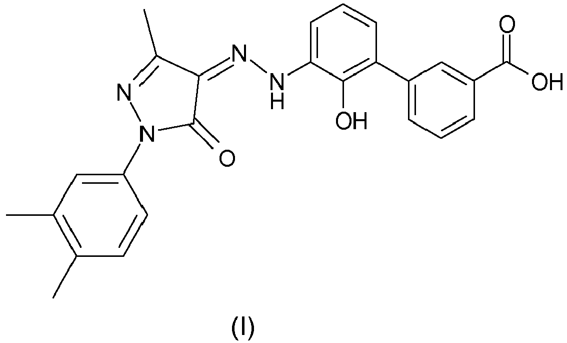 Optionally, the compound of formula (I) may be reacted with an appropriate pharmaceutically acceptable base to yield a pharmaceutically acceptable salt of eltrombopag.

The compound 2'-(benzyloxy)-3'-nitro-1,1'-biphenyl-3-carboxylic acid of formula (VI) or its salts are new intermediate compounds and form part of the invention.

The compound 3'-{(2E)-2-[1-(ethoxycarbonyl)-2-oxopropylidene]hy-drazino}-2'-hydroxy-1,1'-biphenyl-3-carboxylic acid of formula (II) or its salts are new intermediate compounds and form part of the invention.

### DETAILED DESCRIPTION OF THE INVENTION

The term "reflux temperature" as disclosed herein refers to the temperature where the mixture boils in circumstances such that the vapor returns to the stock of liquid after condensing.

The term "room temperature" as disclosed herein refers to a temperature of the environment, without heating or cooling, and is generally comprised of from 20 to 25°C.

For the purposes of the invention, any ranges given include both the lower and the upper end-points of the range. Ranges given, such as temperatures, times, and the like, should be considered approximate, unless specifically stated.

The 2-(benzyloxy)-1-bromo-3-nitrobenzene (compound of formula (VII)) which is one of the starting materials for the preparation of the compound of formula (VI) may be prepared as disclosed in WO 2009005998 (ex. 41a, 94%) or A. Akwabi-Ameyaw et al., Bioorganic & Medicinal Chemistry Letters 2009, 19(16), 4733-4739.

As mentioned above, the compound of formula (VI) may be prepared by reaction of the compound of formula (VII) with a compound of formula (VIII) or a salt thereof or a (C₁-C₄)-alkyl ester thereof, in the presence of Pd(OAc)₂, tricyclohexylphosphine, a base, an appropriate solvent, and at an appropriate temperature.

When appropriate, the ester compound obtained is submited to a hydrolysis reaction with an acid or a base and a suitable solvent to yield the corresponding compound of formula (VI) as free acid or as a salt thereof.

Several boronic compounds of formula (VIII) as well as their esters are commercial compounds. The preparation of the boronic compounds may also be carried out as described, for instance, in Dennis G. Hall. Editor; Boronic Acids - Preparation, Applications in Organic Synthesis and Medicine, 2nd Edition, Wiley-VCH:Weinheim (Germany), 2011, ISBN 9783527325986, chapter I: Properties and preparation of boronic acid derivatives, pages 1-131.

In a preferred embodiment of the preparation process, compound of formula (VIII) is that where Y₁ and Y₂ are independently selected from the group consisting of: hydroxy, methoxy, ethoxy and phenoxy.

In another preferred embodiment, the process is carried out with a compound of formula (VIII) or a salt thereof.

In a more preferred embodiment, the process is carried out with a compound of formula (VIII) where Y₁ and Y₂ are hydroxy, having the formula (Vllla).

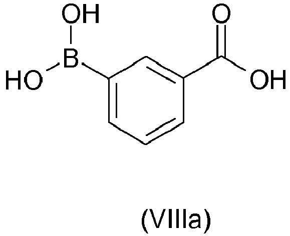

In another particular embodiment of the preparation process, compound of formula (VIII) is that where Y₁ and Y₂ together with the boron atom form a cyclic structure, wherein C is selected from CH₂C(CH₃)₂CH₂ and C(CH₃)₂C(CH₃)₂.

In a preferred embodiment, the base is selected from sodium hydroxide, sodium carbonate, and cesium carbonate.

In a more preferred embodiment of the process, the base is selected from sodium hydroxide and cesium carbonate and the solvent is selected from a (C₄-C₅)-cycloalkyl ether and a (C₄-C₆)-alkyl ether.

In a particular embodiment, first a mixture of the compound of formula (VII), Pd(OAc)₂, tricyclohexylphosphine, the base, and the (C₄-C₅)-cycloalkyl ether is heated at a temperature of from 60 °C to reflux temperature of the mixture, before adding the compound of formula (VIII) or a salt thereof. In another particular embodiment, first all the components are mixed and then heated the reaction mixture to the desired temperature.

Generally, the palladium catalyst is present in an amount comprised of from 0.5 to 10% molar, preferably, between 0.5 y 1.0% molar. Generally, the tricyclohexylphosphine ligand is also present in catalytic amounts with respect to the catalyst, preferably, in an amount of between 100-400% molar, preferably, 200% molar.

In a preferred embodiment, the base is aqueous solution of sodium hydroxide.

In another preferred embodiment, the (C₄-C₅)-cycloalkyl ether is 1,4-dioxane. In another preferred embodiment the (C₄-C₆)-alkyl ether is ethylene glycol diethyl ether.

Generally, the reaction is carried out at a temperature of from 60 °C-reflux temperature of the mixture.

In a particular embodiment, the reaction is carried out at a temperature of 90 °C. In another particular embodiment, the reaction is carried out at reflux temperature. Also generally the reaction time is comprised of from 1 to 24 hours, preferably, from 2 to 6 hours.

The compound of formula (VI) may be isolated in form of free acid or in form of a salt thereof. The salt can be isolated directly from the reaction medium or can be prepared from the isolated free acid by reaction with an appropriate base. Generally, the free acid is isolated from the reaction medium after acidifying the medium with an appropriate acid.

The compound of formula (VI) may be submitted to a hydrogen atmosphere in the presence of palladium catalyst and an appropriate solvent, to yield a compound of formula (IV). In these conditions both the reduction of the nitro group to the amino group and the deprotection of the hydroxyl group of the compound of formula (VI) occur. The reaction may be carried out in the presence of a base such as sodium hydroxide.

In a preferred embodiment, the palladium catalyst is Pd/C.

In a preferred embodiment, the solvent is a (C₁-C₆) alcohol. In a more preferred embodiment, the solvent is methanol.

In a preferred embodiment, this step is carried out at room temperature.

In another preferred embodiment, this reaction step is carried out at a pressure of 0-4 bars, preferably, at a pressure of 4 bars.

The compound of formula (IV) can be isolated from the reaction medium in form of free acid or in from of free base, directly from the reaction medium or by adding an acid or a base depending on the reaction conditions. The compound obtained can be converted into a salt thereof by reaction with an appropriate base or acid. In a particular embodiment, the salt is the hydrochloride salt. The processes of the invention can also be carried out without isolating this intermediate from the reaction medium.

Eltrombopag or its salts may be prepared by a process comprising the steps for the preparation of the compound of formula (IV) from the compound of formula (VII) disclosed above, followed by a step comprising reacting the compound of formula (IV) or a salt thereof with hydrochloric acid, a solvent selected from water or a (C₁-C₄) alcohol, preferably methanol or ethanol, and sodium nitrite to form the corresponding diazonium derivative. This process may be carried out without isolating compound (IV) from the reaction medium. Further, it may be added sulfamic acid. Generally this reaction is carried out at low temperatures, in particular from 0 °C to 5 °C. Generally, the subsequent reaction with compound of formula (IX) is carried out in the presence of a base, preferably an organic base such as triethylamine. An inorganic base can also be used, for instance, sodium bicarbonate. Preferably, the reaction temperature is comprised of from 0 °C to room temperature. Generally, the compound of formula (I) is isolated from the reaction medium in form of free acid by addition of an acid to the reaction medium. It can be converted into a salt thereof by reaction with an appropriate base.

Alternatively, the compound of formula (IV) or a salt thereof (isolated or not from the previous step) may be reacted first with sodium nitrite and then with ethyl acetoacetate to yield a compound of formula (II). Generally, this step is carried out by combining the compound of formula (IV) with hydrochloric acid, a solvent selected from water or a (C₁-C₄) alcohol, preferably methanol or ethanol, and sodium nitrite. Further, it may be added sulfamic acid. Generally this reaction is carried out at low temperatures, in particular from 0 °C to 5 °C. Generally, the subsequent reaction with ethyl acetoacetate is carried out in the presence of a base, preferably an organic base such as triethylamine. Preferably, the reaction temperature is comprised of from 0 °C to room temperature. Generally, the compound of formula (II) is isolated from the reaction medium in form of free acid by addition of an acid to the reaction medium. It can be converted into a salt thereof by reaction with an appropriate base.

Finally, the compound of formula (II) or a salt thereof may be reacted with a compound of formula (III), which is (3,4-dimethylphenyl)hydrazine, or a salt thereof, such as the hydrochloride salt, in the presence of an acid and an appropriate solvent to yield eltrombopag free acid of formula (I).

In a preferred embodiment, the acid is selected from acetic acid and p-toluensulfonic acid. In another preferred embodiment, the solvent is (C₄-C₆)-alkyl or (C₄-C₅)-cicloalkyl ether. Solvents such as N.N-dimethylformamide also yield good results.

In a more preferred embodiment, the ether is tetrahydrofuran. In another preferred embodiment, this step is carried out at reflux temperature of the mixture.

Generally, the eltrombopag is isolated from the reaction medium in form of free acid. If desired, it can be recrystallized for instance using a mixture of isopropanol/water.

The eltrombopag free acid obtained from the process disclosed above may be converted into a pharmaceutically acceptable salt thereof, in particular, to any salt formed from pharmaceutically acceptable non-toxic bases including inorganic or organic bases. There is no limitation regarding the salts, except that if used for therapeutic purposes, they must be pharmaceutically acceptable.

In a preferred embodiment, the base is ethanolamine and the salt of eltrombopag obtained is the olamine salt.

As some of the intermediates to prepare the compound of formula (I) are also in form of free acid compounds, salts may be prepared from non-toxic bases, including inorganic and organic bases. Examples of inorganic bases include sodium hydroxide, sodium carbonate, sodium methoxide, or sodium tert-butoxide. Examples of organic bases are for instance, ethanolamine.

Some intermediate to prepare the compound of formula (I) as the intermediate (IV) can also be in form of salts prepared from non-toxic acids, including, inorganic or organic acids. Such acids include for instance acetic, benzenesulfonic, benzoic, camphorsulfonic, citric, ethansulfonic, fumaric, gluconic, glutamic, hydrobromic, hydrochloric, lactic, maleic, malic, mandelic, methanesulfonic, phosphoric, succinic, sulfuric, tartaric, or p-toluensulfonic acid.

The preparation of salts of the compounds of formula (I) or of the intermediates that intervene in the process of the invention, in particular, compounds (VIII), (VI), (IV), and (II) can be carried out by methods known in the art. For instance, they can be prepared from the parent compound, which contains an acidic moiety, by conventional chemical methods. Generally, such salts are, for example, prepared by reacting the free acid forms of these compounds with a stoichiometric amount of the appropriate pharmaceutically acceptable base in an appropriate solvent. When an acid salt is desired then the compound is reacted with the corresponding pharmaceutically acceptable acid.

With regard to the specific conditions for carrying out the steps of the process of the invention, the skilled person would know how to adjust the parameters of each of the steps indicated above in the light of the description and examples of the present invention.

It is also part of the present invention the provision of single reaction steps of the process to obtain the compound of formula (I) from the compound of formula (VII), as well as the combination of two of more sequential steps of the process described above that goes through the formation of the pyrazole ring in two steps, the process being able to be carried out in sequential steps isolating the intermediates obtained, or alternatively, in one pot, without isolation of intermediate compounds.

In a particular embodiment, the preparation process comprises the preparation of compound of formula (IV) from the compound of formula (VI) optionally in combination with one or more features of the several preferred or particular embodiments defined above for this step.

In another particular embodiment, the preparation process comprises the preparation of compound of formula (IV) from the compound of formula (VII), i.e. two process steps, optionally in combination with one or more features of the several preferred or particular embodiments defined above for these two steps.

In another particular embodiment, the preparation process comprises the preparation of eltrombopag of formula (I) from the compound of formula (II), optionally in combination with one or more features of the several preferred or particular embodiments defined above for this step.

In another particular embodiment, the preparation process comprises the preparation of eltrombopag of formula (I) from the compound of formula (IV), i.e. two process steps, optionally, in combination with one or more features of the several preferred or particular embodiments defined above for these two steps.

In another particular embodiment, the preparation process comprises the preparation of compound of formula (I) or its salts from the compound of formula (VI).

As mentioned above, the compound of formula (VI) and the compound of formula (II) are new intermediates and form part of the invention. The new intermediates that intervene in the preparation process of the invention may be in crystalline form either as free solvation compounds or as solvates (e.g. hydrates) and it is intended that both forms are within the scope of the present invention. Methods of solvation are generally known within the art. In a preferred embodiment, the compound of formula (I) is a hydrate.

It is considered part of the invention, the use of the compound of formula (VI) as an intermediate to prepare eltrombopag of formula (I) or a salt thereof. It is also considered part of the invention the use of the compound of formula (II) as an intermediate to prepare eltrombopag of formula (I) or a salt thereof.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1: Preparation of 2'-(benzyloxy)-3'-nitro-1,1'-biphenyl-3-carboxylic acid

A mixture of 2-(benzyloxy)-1-bromo-3-nitrobenzene (800 mg, 2.59 mmol), Tricyclohexylphosphine (145 mg, 0.51 mmol), Pd(OAc)₂ (58 mg, 0.25 mmol) and NaOH 1M (5.1 mL, 5.18 mmol) was refluxed in 1,4-dioxane for 15 min. After cooling down to room temperature 3-carboxyphenylboronic acid (472 mg, 2.84 mmol) was added and the reaction mixture refluxed again for 4 h. The mixture was cooled to room temperature, filtered through a pad of Celite® and the pad was washed with EtOAc (30 mL). The filtrate was washed with HCl 15% and water and the organic layer was dried over sodium sulfate, filtered and concentrated to give a brown solid. The solid was purified in EtOAc at 0/5 °C to obtain 2'-(benzyloxy)-3'-nitro-1,1'-biphenyl-3-carboxylic acid as a brown solid (700 mg, 77%).
Alternatively, it is purified directly by crystallization in ethanol.

### Example 2: Preparation of 2'-(benzyloxy)-3'-nitro-1,1'-biphenyl-3-carboxylic acid

To a desoxigenated solution of 2-(benzyloxy)-1-bromo-3-nitrobenzene (7.12 g, 23.11 mmol), 3-Carboxyphenylboronic acid (4.22 g, 25.42 mmol), Tricyclohexylphosphine (0.129 g, 0.46 mmol) and NaOH 10% (18.50 mL) in 1,4-dioxane (70 mL) was added Pd(OAc)₂ (0.052 g, 0.23 mmol) and the mixture was refluxed for 7 h. After cooling down to room temperature, EtOAc (150 mL), HCl 5% (50 mL) and brine (100 mL) were added and the organic layer was dried over sodium sulfate, filtered and concentrated. The crude was purified by flash chromatography over silica (MeOH/CH2Cl2; gradient 0:100 to 5:95) to give 2'-(benzyloxy)-3'-nitro-1,1'-biphenyl-3-carboxylic acid as a greenish yellow solid (6.4 g, 79%). Purity by HPLC: 98.3%a/a. HPLC conditions: XBridge HILIC 3.5 um, 4.6 x 150 mm, flow rate: 1.2 ml/min; Acetonitrile: water: ammonium acetate buffer: 12 min in 95:0:5 + 95:0:5 to 60:35:5 in 15 min + 8 min in 60:35:5; sample in acetonitrile/methanol).

Alternatively, it is purified directly by crystallization in isopropyl alcohol.

### Example 3: Preparation of 2'-(benzyloxy)-3'-nitro-1,1'-biphenyl-3-carboxylic acid

A mixture of 2-(benzyloxy)-1-bromo-3-nitrobenzene (80 mg, 0.259 mmol), carboxyphenylboronic acid (45 mg, 0.27 mmol), Cs₂CO₃ (183 mg, 0.518 mmol), Tricyclohexylphosphine (0.014 g, 0.05 mmol) and 1,4-dioxane was desoxigenated with Ar and then Pd(OAc)₂ (3 mg, 0.012 mmol) was added. The mixture was heated at 90 °C for 24 h and then cooled down to room temperature, filtered through a pad of Celite® and the pad was washed with EtOAc. The filtrate was washed with water, HCl 15% and water, and was dried over sodium sulfate, filtered and concentrated to give a brown solid. The crude was purified by flash chromatography over silica (MeOH/CH₂Cl₂; 6:94) to give 2'-(benzyloxy)-3'-nitro-1,1'-biphenyl-3-carboxylic acid as a brown solid (83 mg, 81 %)

### Comparative Example 1: Preparation of 2'-(benzyloxy)-3'-nitro-1,1'-biphenyl-3-carboxylic acid

It were reproduced the reaction conditions disclosed in Example 3c of WO200189457A2 but starting from the benzyloxy protected derivative instead the methoxy protected derivative.

To a mixture of 2-(benzyloxy)-1-bromo-3-nitrobenzene (247 mg, 0.802 mmol), 3-Carboxyphenylboronic acid (140 mg, 0.842 mmol) in 1,4-dioxane (4.0 mL) was added Na₂CO₃ aq. 2M (0.80 mL) and the mixture was desoxigenated with Ar. Tetrakis(triphenylphosphine)palladium(0) (37 mg, 0.032 mmol) was added and the mixture was refluxed for 21 h. The reaction was cooled down to room temperature, EtOAc and HCl 10% were added and the mixture was stirred for 5 minutes. Organic layer was washed with HCl 10% and water, dried over sodium sulfate, filtered and concentrated. The crude was purified by flash chromatography over silica (MeOH/CH₂Cl₂; gradient 0:100 to 3:97) to give an orange oil which was purified in Et₂O/hexanes to give 2'-(benzyloxy)-3'-nitro-1,1'-biphenyl-3-carboxylic acid as a yellow solid (104 mg, 37%) contaminated with triphenylphosphine oxide. Purity by HPLC: 82.43%a/a; 6.03% POPh₃ a/a.HPLC conditions: SunFire C18 5 um, 2.1 x50 mm, flow rate: 0.3 mL/min; Acetonitrile and methanol 1:1: water: ammonium acetate buffer 2 min in 10:85:5 + 10:85.5 to 95:0:5 in 6 min + 7 min in 95:0:5; sample in methanol).

### Comparative Examples 2: Preparation of 2'-(benzyloxy)-3'-nitro-1,1'-biphenyl-3-carboxylc acid

It was prepared 2'-(benzyloxy)-3'-nitro-1,1'-biphenyl-3-carboxylic acid in the conditions disclosed by M. H. Yates et al., in Org. Process Research & Dev., 2009, vol. 13, p.268-275, i.e. Pd/C as catalyst, K₂CO₃ as base, t-Butanol as solvent, at a temperature of 80 °C maintained overnight.

According to thin layer chromatography, No formation of the final product is observed, being the starting material the majority product.

### Comparative Examples 3: Preparation of 2'-(benzyloxy)-3'-nitro-1,1'-biphenyl-3-carboxylic acid

It was prepared 2'-(benzyloxy)-3'-nitro-1,1'-biphenyl-3-carboxylic acid in the conditions disclosed in by Shuji Jinno et al., in Bioorg. Med. Chem. Lett 1999, vol. 9, 1029-1032, i.e. Pd(OAc)₂ as catalyst, K₂CO₃ as base, water as solvent, in the presence of TBAB, at a temperature of 80 °C maintained overnight.

According to thin layer chromatography, being the starting material the majority product (TLC conditions for compound Vil: EtOAc/hexane 1:9; TLC conditions for compound VI: CH₂Cl₂/MeOH 9:1).

### Comparative Examples 4: Preparation of 2'-(benzyloxy)-3'-nitro-1,1'-biphenyl-3-carboxylic acid

It was prepared 2'-(benzyloxy)-3'-nitro-1,1'-biphenyl-3-carboxylic acid in the conditions disclosed by S. K. Fehler et al., in Tetrathedron lett. 2012, vol. 53, pp. 2189-2194, i.e. Pd(dbpf)Cl₂ as catalyst, K₂CO₃ as base, MECN/water as solvent at a temperature of 60 °C during 17 hours.

According to HPLC/MS after 17 hours there was a 40.6% of final product in front of 44.1% of starting material (HPLC-MS conditions: SunFire C18 5 µm, 2.1x50 mm; flow rate: 0.3 mL/min; gradient A:B 2 min en 10:85:5 + 6 min de 10:85.5 a 95:0:5+ 7 min en 95:0:5; A: CH₃CN:MeOH (1:1); B: water; C: ammonium acetate, 100 mM pH 7; sample in methanol).

### Example 4: Preparation of 3'-amino-2'-hydroxy-1,1'-biphenyl-3-carboxylic acid

A mixture of 2'-(benzyloxy)-3'-nitro-1,1'-biphenyl-3-carboxylic acid (270 mg, 0.777 mmol) and Pd/C (paste 10%, 170 mg) in MeOH (10 mL) was stirred under hydrogen atmosphere overnight at room temperature. The reaction mixture was filtered through a pad of Celite® and the pad was washed with MeOH. The filtrate was concentrated to give 3'-amino-2'-hydroxy-1,1'-biphenyl-3-carboxylic acid as a brown solid (235 mg, 89%).

### Example 5: Preparation of 3'-amino-2'-hydroxy-1,1'-biphenyl-3-carboxylic acid

A solution of 2'-(benzyloxy)-3'-nitro-1,1'-biphenyl-3-carboxylic acid (2.0 g 5.725 mmol) in metanol (32 ml), water (8 ml) and 10% aquous sodium hydroxide (2.34 ml, 5.840 mmoles) was hydrogenad over 5% palladium on carbon (200 mg) at 20 °C and 4.0 bars for 2 hours. To the reaction mixture 35% aquous hydrochloric acid (1.76 ml, 20.15 mmoles) was added and the solution was filtered through a pad of Celite®. This solution was directly used for the next step.

### Example 6: Preparation of 3'-{(2E)-2-[1-(ethoxycarbonyl)-2-oxopropylidene]hy-drazino}-2'-hydroxy-1,1'-biphenyl-3-carboxylic acid

To 3'-amino-2'-hydroxy-1,1'-biphenyl-3-carboxylic acid (715 mg, 3.11 mmol) in MeOH (10 mL) was added HCl aq. 4.1M (0.39 mL, 3.11 mmol) and the mixture was stirred for 30 min and then cooled to 0/5 °C. A solution of NaNO₂ (214 mg, 3.11 mmol) in water (0.61 mL) was added and then the mixture stirred for I h. A solution of sulfamic acid (33 mg, 0.34 mmol) in water (0.61 mL) was added and the reaction was stirred for 2 h. Triethylamine (5 mL) was added to reach pH 7-8, then ethyl acetoacetate (0.39 mL, 3.11 mmol) was added and the reaction was warmed to room temperature and stirred overnight. Water was added and then HCl 15% was added dropwise until pH 4. The mixture was filtered to give 3'-{(2E)-2-[1-(ethoxycarbonyl)-2-oxopropylidene]hy-drazino)-2'-hydroxy-1,1'-biphenyl-3-carboxylic acid as an orange solid (886 mg, 77%).

### Example 7: Preparation of 3'-{(2E)-2-[1-(ethoxycarbonyl)-2-oxopropylidene]hy-drazino}-2'-hydroxy-1,1'-biphenyl-3-carboxylic acid

To a mixture of 3'-amino-2'-hydroxy-1,1'-biphenyl-3-carboxylic acid (100 mg, 0.44 mmol) in water (0.44 mL) cooled at 0/5 °C was added HCl 15% (0.29 mL). A solution of NaNO₂ (30 mg, 0.44 mmol) in water (0.30 mL) was added and then the mixture stirred for I h. A solution of ethyl acetoacetate (0.06 mL, 0.44 mmol) in EtOH/water 1:1 (0.88 mL) was added and then triethylamine (0.80 mL) was added and the mixture stirred at 0/5 °C for 2 h. The reaction mixture was warmed to room temperature and stirred for 1.5 h. Water was added and then HCl 15% was added dropwise until pH 2. The mixture was filtered to give 3'-{(2E)-2-[1-(ethoxycarbonyl)-2-oxopropylidene]hy-drazino}-2'-hydroxy-1,1'-biphenyl-3-carboxylic acid as an orange solid (137 mg, 85%).

### Example 8: Preparation of Eltrombopaa

To a mixture of 3'-{(2E)-2-[1-(ethoxycarbonyl)-2-oxopropylidene]hy-drazino}-2'-hydroxy-1,1'-biphenyl-3-carboxylic acid (400 mg, 1.08 mmol) and (3,4-dimethylphenyl)hydrazine hidrochloride (281 mg, 2.16 mmol) in THF (8 mL) was added AcOH (0.18 mL, 3.24 mmol) and the mixture was refluxed for 6 h. Then (3,4-Dimethylphenyl)hydrazine hidrochloride (28 mg, 0.21 mmol) was added and the mixture was refluxed overnight. The reaction mixture was cooled down to room temperature and concentrated. The raw material was treated with iPrOH (20 mL), refluxed for 20 minutes and cooled down slowly to room temperature. Water (12 mL) was added and the mixture filtrated to give Eltrombopag as an orange solid (382 mg, 80%).

### Example 9: Preparation of Eltrombopaa monohydrate

A solution of 3'-amino-2'-hydroxy-1,1'-biphenyl-3-carboxylic acid hydrochloride salt (1.31 g 5.725 mmol) in methanol water, obtained as described in Example 4', was cooled to 0-5 °C and a solution of sodium nitrite (0,403 g, 5.840 mmol) in water (2 ml) was added over 15 minutes. The reaction was stirred at 0-10 °C for one hour and then it was heated to 20 °C. Triethylamine (about 2,5 ml) was added to bring the pH to 8-9 and 1-(3,4-dimethylphenyl)-3-methyl-1 H-pyrazol-5-ona (1.16 g, 5,725 mmol) was added in one portion. The mixture was stirred for 2 hours at 20 °C maintaining the pH 8-9. Hydrochloric acid (4M, about 4 ml) was added to adjust the pH to 1.5-2.0. The precipitate was filtered, washed with water and dried at 40 °C to yield 2.492 g (93%).

### REFERENCES CITED IN THE APPLICATION

- WO200189457A2
- WO2010114943A1
- EP2152811A1
- WO2009005998A1
- A. Akwabi-Ameyaw et al., Bioorganic & Medicinal Chemistry Letters 2009, 19(16), 4733-4739
- M. H. Yates et al., Org. Process Research & Dev., 2009, vol. 13, p.268-275
- Shuji Jinno et al., Bioorg. Med. Chem. Lett 1999, vol. 9, 1029-1032
- S. K. Fehler et al., Tetrahedron lett. 2012, vol. 53, pp. 2189-2194
- Dennis G. Hall. Editor; Boronic Acids - Preparation, Applications in Organic Synthesis and Medicine, 2nd Edition, Wiley-VCH:Weinheim (Germany), 2011, ISBN 9783527325986, chapter I: Properties and preparation of boronic acid derivatives, pages 1-131.

## Claims

1. A preparation process of a compound of formula (VI) or a salt thereof, 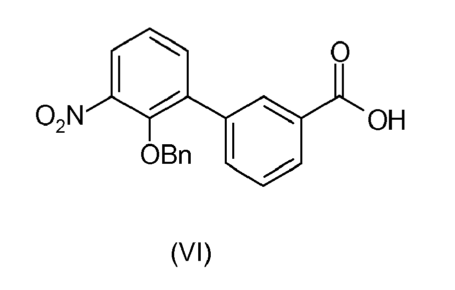 which comprises reacting a compound of formula (VII) 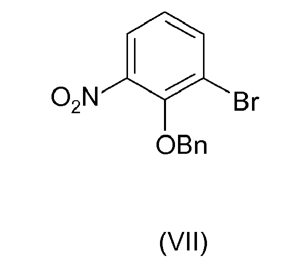 with either a compound of formula (VIII) or a salt thereof or a (C₁-C₄)-alkyl ester thereof, 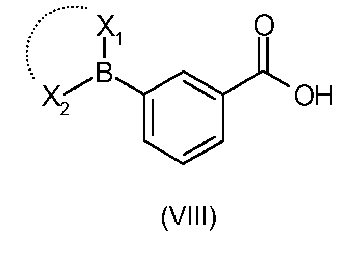 in the presence of Pd(OAc)₂, tricyclohexylphosphine, a base, an appropriate solvent, and at an appropriate temperature; if appropriate, submitting the compound thus obtained to a hydrolysis reaction; and isolating the compound thus obtained in form of a salt of compound of formula (VI) or in form of the free acid by adding an acid;
wherein:
Bn is a benzyl group;
X₁ and X₂ are each independently selected from the group consisting of hydroxy, (C₁-C₄)-alcoxy and phenoxy, the latter optionally substituted by a (C₁-C₄)-alcoxy or a (C₁-C₄)-alkyl; or alternatively X₁ and X₂ can be taken together with the boron atom to form a cyclic structure selected from the following ones, 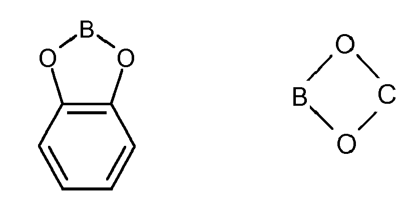
C is (CH₂)ₙ; wherein the hydrogen atoms of the CH₂ group are optionally replaced by a substituent selected from (C₁-C₄)-alkyl, phenyl and mono- or disubstituted phenyl, the substituents being (C₁-C₄)-alkyl and (C₁-C₄)-alkoxy, and;
n is an integer from 2 to 4.

2. The preparation process according to claim 1, wherein the compound of formula (VIII) is the compound of formula (Villa)

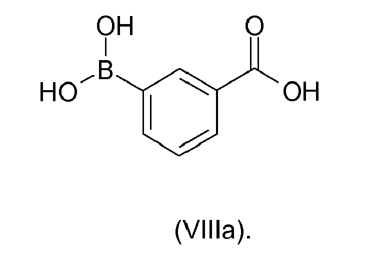

3. The preparation process according to any of the claims 1-2, wherein the base is selected from sodium hydroxide and cesium carbonate and the solvent is selected from a (C₄-C₅)-cycloalkyl ether and a (C₄-C₆)-alkyl ether.

4. The preparation process according to any of the claims 1-3, wherein the base is sodium hydroxide.

5. The preparation process according to any of the claims 1-4, wherein the (C₄-C₅)-cycloalkyl ether is 1,4-dioxane.

6. The preparation process according to any of the claims 1-5, wherein the temperature is a temperature of from 60 °C to reflux temperature of the mixture.

7. The process according to any of the claims 1-6, further comprising submitting the compound of formula (VI), in form of free acid or as a salt thereof, to a hydrogen atmosphere in the presence of an appropriate palladium catalyst and an appropriate solvent, to yield a compound of formula (IV), and, optionally, reacting the compound of formula (IV) with an appropriate base or acid to yield a salt of the compound of formula (IV).

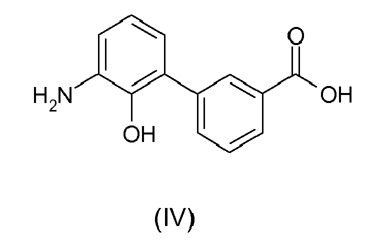

8. The preparation process according to claim 7, wherein the palladium catalyst is Pd/C.

9. The preparation process according to any of the claims 7-8, further comprising reacting the compound of formula (IV) or a salt thereof, first with sodium nitrite and then with ethyl acetoacetate to yield a compound of formula (II), and optionally reacting the compound of formula (II) with an appropriate base to yield a salt of the compound of formula (II).

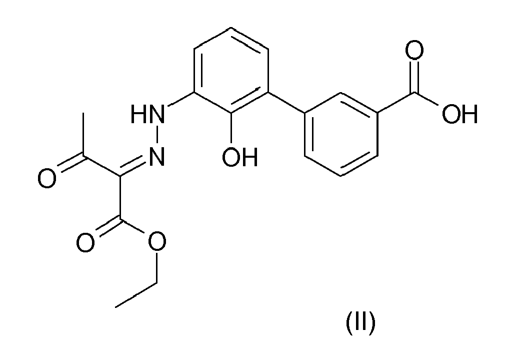

10. The preparation process according to claim 9, further comprising reacting a compound of formula (II) with a compound of formula (III) or a salt thereof, in the presence of an acid and an appropriate solvent 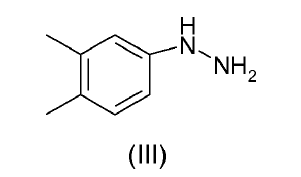 to yield eltrombopag free acid of formula (I) and, 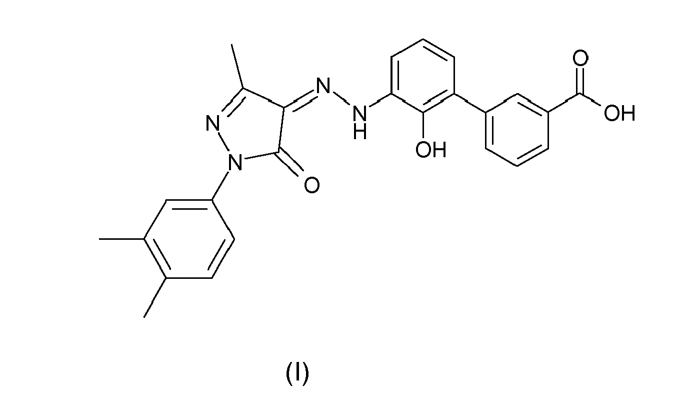 optionally, reacting the compound of formula (I) with an appropriate pharmaceutically acceptable base to yield a pharmaceutically acceptable salt of eltrombopag.

11. The preparation process according to claim 10, wherein the acid is acetic acid.

12. The preparation process according to any of the claims 10-11, wherein the ether is tetrahydrofuran.

13. The preparation process according to any of the claims 10-11, wherein the base is ethanolamine and the salt of eltrombopag obtained is the olamine salt.

14. The preparation process according to any of the claims 7-8, further comprising reacting the compound of formula (IV) or a salt thereof with a compound of formula (IX), 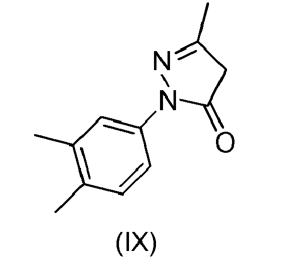 to yield eltrombopag free acid of formula (I) and, optionally, reacting the compound of formula (I) with an appropriate pharmaceutically acceptable base to yield a pharmaceutically acceptable salt of eltrombopag.

15. A compound of formula (VI) or a salt thereof
